# EUROPEAN PATENT SPECIFICATION

(11) **EP 0 438 043 B1**
(45) Date of publication and mention of the grant of the patent: **29.05.1996**
(21) Application number: 90850426.9
(22) Date of filing: 21.12.1990
(51) Int. Cl.: A61F 2/16

(54) **Intraocular lens with high strength haptics**
Intraoculare Linse mit Haltebügeln hoher Festigkeit
Lentille intraoculaire avec agrafes effilées à grande résistance

(30) Priority: 19.01.1990 US 467789
(43) Date of publication of application: 24.07.1991
(73) Proprietor: Pharmacia AB, S-171 97 Stockholm (SE)
(72) Inventor: Krug, John A., Orange, CA 92665 (US); Schwartz, Alan, Costa Mesa, CA 92667-1462 (US); Healy, Donald D., Duarte, CA 91010 (US); Yang, Stan, San Gabriel, CA 91776 (US); Koenig, Don, Temple City, CA 91780 (US)
(74) Representative: Widén, Björn

(56) References cited:
- DE-A- 2 748 579
- DE-A- 3 627 488
- DE-A- 3 635 111
- US-A- 4 781 718
- US-A- 4 787 903

## Description

### Background of the Invention

The present invention relates to intraocular lenses and more particularly concerns intraocular lenses in which the lens optic and/or the supporting haptics have significantly increased strength, flexibility, and resiliency.

Among the procedures for treating cataracts in the human eye is replacement of the clouded damaged natural lens with a plastic intraocular lens. Some exemplary configurations of intraocular lenses and their haptics are shown in the U. S. Patents to Feaster, 4,842,600, Sheets, 4,813,953, Walman, 4,504,981, Burk, 4,504,982, Cozean, Jr., 4,441,217, Kelman, 4,451,938, Lopez, 4,494,254, Arnott, 4,476,591, Anis, 4,804,361, and Jaffe, 4,822,358. All of these patents, except the patent to Burk, show intraocular lenses with different types of long slender haptics which may be formed integrally with the optic or lens portion of the device.

The intraocular lens is generally implanted, often during the same procedure in which the clouded lens is removed, in either the anterior or posterior chamber of the eye. The intraocular lens, which includes a portion termed the "optic", which is the lens portion itself, and supporting legs or loops termed "haptics", is introduced into in the eye through a small incision and then appropriately positioned within the eye itself. For example, to place a lens in the posterior chamber of the eye, where the lens has an inferior and superior haptic, the inferior haptic is first passed through the pupil and into the posterior chamber. The superior haptic is then grasped with a suitable instrument and compressed or bent to a position close to the optic and pushed into the posterior chamber with the optic while held in this compressed position. Thereafter, the superior haptic is released and the lens is then finally positioned, to be held in place by engagement of the haptics with the eye tissue. Thus it will be seen that flexibility and resiliency of the haptics is desirable to facilitate at least the described type of implantation of the intraocular lens.

In addition, such flexibility is highly desirable for proper support of the optic after implantation. A sudden motion of the head or a blow to the head may tend to move the intraocular lens relative to the supporting tissue of the eye, thus causing the haptics to place increased strain on supporting tissue. The greater the stiffness of the haptic, the greater the possibility that repetition of such motions or blows will cause the haptic to damage the tissue.

Accordingly, to accommodate the sometimes severe bending which may be experienced by the haptics during implantation of the lens, and to minimize adverse interaction of the haptics and eye tissue during various motions of the head after implantation, it is highly desirable that the intraocular lens have haptics that are strong, flexible and resilient, yet are sufficiently stable to provide secure placement of the lens within the eye. This has not been possible heretofore because of the limited types of materials available and approved for such use.

Polymethylmethacrylate (PMMA), or co-polymers thereof, are widely used for making intraocular lenses, largely because of two important considerations. These are, first, the fact that the material is inert and will not react with the tissue of the eye, and, second, the fact that the materials have been approved by the Federal Food and Drug Administration for such use. However, the PMMA generally employed in the manufacture of intraocular lenses, because it is relatively inflexible, may not react as well as desired to the relatively large amount of bending required during various lens insertion techniques nor to the stresses imposed thereafter by sudden head motion or impact.

While PMMA subjected to biaxial stretch has been utilized for several purposes, there has been no suggestion to utilize these materials in the production Of high strength intraocular lenses with haptics of increased flexibility. U. S. Patent 4,278,723 discloses the formation of shaped bodies from sheet material biaxially stretched more than 40%, preferably 60- 80% to produce a more corrosion-resistant and weather-resistant product. U. S. Patent 4,228,267 discloses a process for stretching PMMA film to produce scratch-resistant optical articles.

The common intraocular lens known in the art has an optic disc on the order of about 6mm in diameter, with the haptics extending outwardly therefrom to provide a total outside diameter in the range of 9 to 14mm. The haptics are typically long, slender elements having cross sectional diameters on the order of 0.1mm to 0.18mm when round or similar dimensions per side when polygonal or elliptical in shape and generally radially extending from the optic for several millimeters. Lacking great strength and flexibility, these hair-like supporting structures may not withstand bending or repetitive flexing. Some repetitive bending may be encountered in the implantation procedure itself if the operating surgeon should release his instrument's grip on the superior haptic after it has been bent toward the optic, thereby requiring a second or possibly still further major bending of this element.

Because the PMMA material previously used for intraocular lenses has a relatively high rigidity, it is highly desirable to improve its strength, flexibility and resiliency in order to decrease the possibility of fracture and to improve the positioning of the lens in the eye.

Accordingly, it is an object of the present invention to provide an intraocular lens which avoids or minimizes the above mentioned problems.

### Summary of the Invention

In carrying out the principles of the present invention in accordance with preferred embodiments thereof, an intraocular lens having an optic body and at least one haptic integral with and extending from the lens body is formed of a material selected from the class of polymethylmethacrylate and co-polymers thereof, which material has been processed by stretching along at least two mutually angulated axes, to provide highly oriented polymer chains and increased tensile strength and flexibility, whereby the lens haptic exhibits unexpectedly increased strength, flexibility, and resiliency.

### Brief Description of the Drawings

Figure 1 is a front view of a typical intraocular lens including optic and haptic made of a material embodying principles of the present invention;
Figure 2 is a side view of the lens of Figure 1;
   and
Figure 3 shows a blank from which a lens is to be machined.

### Detailed Description of the Invention

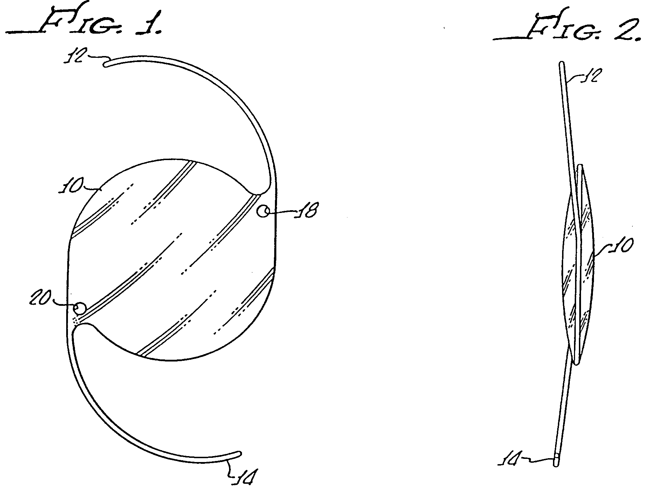

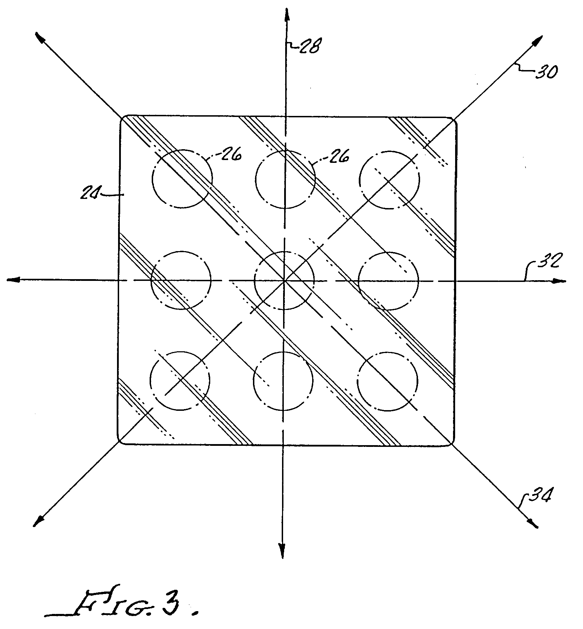

Many different intraocular lens configurations and sizes may be employed in the present invention. For purposes of exposition, a typical and exemplary intraocular lens configuration is illustrated in Figure 1, which shows an optic or lens disc 10 suitably shaped for proper focusing and having integrally formed supporting elements or haptics 12 and 14 extending therefrom. Exemplary lens 10 may be bi-convex, as shown, or plano-convex, concavo-convex or any other optical configuration as desired. The optic may also have refractive and diffractive optic portions, several refractive curves or an aspheric surface to give bifocal or multifocal capabilities and by be formed of a material having different optical or physical properties than the material forming haptics 12 and 14. Some of the many other configurations of lenses and their haptics, which may be used in practice of the present invention, are shown in the U. S. patents identified above, and those skilled in the art will appreciate that virtually any haptic shape, configuration, or number may be utilized in accordance with the teachings of the present invention. The haptics 12 and 14 of exemplary lens 10 are oppositely directed, curved, partially looped supports emanating from optic 10 at substantially diametrically opposed portions of the optic. While the haptic may have various configurations, as shown in the U. S. patents identified above, it is common for the haptic to form a curve roughly parallel to but spaced from the edge of the haptic. Small apertures 18,20 are provided in the optic adjacent the connection with haptics for reception of implantation tools. However, apertures 18,20 are not essential to the practice of the present invention and may be deleted or positioned in different locations.

In lenses of this type the central portion or optic is a solid body of approximately 6mm in diameter, with the very small cross section haptics extending outwardly to create an overall size, between the free ends of the haptics, of between about 9 and 14mm. Typically each haptic has a generally square cross section that is about 0.15mm on each side. Other configurations and dimensions may also be employed. It will be readily appreciated that any forces exerted on the haptics create high stress at the junction between the haptics and the optic from which the haptics are generally cantilevered. Thus high strength and flexibility are major considerations.

According to principles of the present invention, as carried out in an exemplary embodiment thereof, an intraocular lens of any one of a variety of known configurations is made of a material which, by virtue of its use in an intraocular lens, enables the lens to provide highly desirable qualities of increased strength and flexibility. Instead of being made of a material, such as conventional PMMA which has generally randomly oriented polymer chains, as occurs in the standard condition of the material, lenses of the present invention are made from PMMA or copolymers thereof, as will be more particularly described below, that has been processed to provide highly oriented polymer chains, greatly increasing the strength and flexibility of the material. In general, to obtain these properties, PMMA material in sheet form is stretched along a plurality of different axes so as to provide highly oriented polymer chains and improved strength and flexibility. While not required, the axes along which the material is drawn preferably intersect in the center of the sheet of material. Material processed in this matter has not heretofore been employed in the manufacture of intraocular lenses. Use of such material to make an intraocular lens provides surprising and unexpected improvements in the physical properties of the lens, in the procedures for implantation and in the actual service of such lenses.

Materials which can be employed to provide the unexpectedly high tensile strength, flexibility and resiliency of the intraocular lenses of the present invention include, in addition to polymethylmethacrylate, co-polymers of polymethylmethacrylate, such as butyl acrylate, ethyl acrylate, and lauryl acrylate.

In the processing of material, such as a polymethylmethacrylate, in order to provide a blank from which an intraocular lens is to be machined in accordance with the teaching of the present invention, a sheet of substantially pure, transparent and colorless polymethylmethacrylate is first treated to minimize surface irregularities which may give rise to points of stress concentration during subsequent processing, for example, by buffing, polishing or other known finishing techniques. The material is then covered with a protective cover, such as, for example, by placing the sheet between lint free towels and wrapping it to prevent foreign particle build-up and to protect the polished and buffed surface. The wrapped sheet is then placed in an oven to uniformly heat the material to a temperature of between 35°C and 150°C, said temperature preferably near but below the glass transition temperature of the material. Preferably the sheet material initially has a thickness in the range of from 1/8 of an inch (0.32 cm) to 1 inch (2.54 cm), preferably about 3/8 of an inch (0.95 cm). While the material is uniformly maintained at a fixed temperature within the indicated range, it is clamped in a machine that stretches the heated sheet simultaneously along several different axes in a plane.

It is important that the stretching be done at least bi-axially, that is, along two mutually angulated and preferably mutually orthogonal axes. It is also contemplated that stretching may occur along a larger number of axes, such as, for example, six or more axes which are uniformly angularly spaced from one another. The material is stretched by synchronously pulling along each of the several axes with a force of at least 250 pounds (1110 N) (for a sheet of 3/8 of an inch (0.95 cm) thickness, for example). Stretching may be accomplished by clamping opposite edges in a machine that simultaneously exerts oppositely directed tensile forces on opposite edges of the material in the directions of each of the several axes along which the material is to be stretched. Each tensile force is exerted in an amount sufficient to stretch the material and to increase its dimension along the axis of stretch by an amount in the range of 20% to 65%. Stretching by 40% is presently preferred. Preferably the amount of stretch that is achieved is the same along all of the axes of stretch.

The stretched sheet, still held in its stretched condition by the clamps of the stretching machine, is then cooled at a controlled rate to room temperature (about 21°C to 22°C). This process results in a very high degree of uniformity of orientation of the polymer chains within the plastic sheet material, which imparts to the material greatly increased strength and flexibility.

One or more blanks or buttons are then cut from the stretched and cooled material, at an area thereof that is displaced at least two inches inwardly from the edges to avoid areas of distortion and ripples or clamping stresses at the edges. Preferably, for stretching a material having a thickness in the order of about 3/8 of an inch, a rectangular material sheet, having dimensions before stretching of between 12 and 20 inches on each side is employed. After stretching and cooling the central area of between 8 and 14 inches is used to cut a plurality of blanks for manufacture of a number of intraocular lenses. The stretched blank, which is thicker and larger in all dimensions than the lens to be made therefrom, or a sheet from which a large number of complete lenses and haptics may be machined, is produced using a numerically controlled milling machine and/or lathe, and one or more integral intraocular lenses, including center optic 10 and its integral haptics 12,14, are machined therefrom, as is known in the art.

Without removing or disturbing the machined blanks, all chips, cutting fluids and other foreign matter are preferably removed by means of ultrasonic vibration, and the machined parts are air dried using clean, dry compressed air. The fully machined lenses are then polished until all sharp edges and corners have been removed and properly rounded. After further cleaning, the lenses can be individually covered by wrapping for protection during storage and handling until ready for sterilization and shipping prior to use.

The use of the material processed as described herein, provides the haptics of these lenses with markedly improved tensile strength, on the order of 350 to 600 grams for haptics of the described dimensions. This may be compared with haptics of a conventional polymethylmethacrylate of the same dimensions which exhibit a tensile strength of only approximately 100 to 200 grams, usually in the range of 120 grams. Further, it has been observed that haptics of the stretched PMMA material are considerably more flexible and resilient than haptics made of conventional non-stretched PMMA material. It is found that intraocular lenses made of the stretched materials as described herein have haptics that can be bent as much as 180° to either side through approximately 20 cycles before failure. In a lens made of conventional unstretched PMMA material haptics of the same configuration and dimension will frequently fracture upon being subjected to a single 90° bend. Also all lenses of the same design machined from a sheet of stretched material or different sheets stretched under the same condition have physical properties substantially the same.

It is also contemplated that manufacture of the described intraocular lens may start with a sheet of a thickness greater than one inch (2.54 cm). For example, a five inch (12.7 cm) thick sheet of polymethylmethacrylate is heated, stretched and cooled, substantially as described above, and cylindrical plugs which may be up to five inches (12.7 cm) thick (depending upon the thickness of the starting material and the amount of stretch imparted) are cut from interior portions of the sheet or other portions displaced,inwardly from the edges. These plugs are then cut into slices, each slice forming a blank from which an improved intraocular lens as described herein will be machined.

There have now been described intraocular lenses and their manufacture which provide haptics of unexpectedly great strength, flexibility and resiliency. Such haptics help to minimize motion of the center portion or optic of the lens on sudden motion of the head or a blow to the head, while the greater flexibility of the optics minimizes the possibility of breakage upon a shock or bending during implantation. The increased flexibility not only improves the life and operation of the lens, but also greatly facilitates surgical implantation procedures, which may itself be further facilitated by use of colored haptics provided by the present invention.

Having thus described preferred embodiments of the present invention, it should be noted by those skilled in the art that the disclosures herein are exemplary only and that other alternatives, adaptations, and modifications may be made within the scope of the present invention. Thus, by way of example and not of limitation, it is contemplated that alternative lens configurations may be used other than the specific optic and haptic configurations as shown. Accordingly, the present invention is not limited to the specific embodiment illustrated herein.

## Claims

1. An intraocular lens including a transparent optic body (10) defining a plane and at least one haptic member (12, 14) fixed to and extending outwardly from said optic body,
said at least one haptic member being integral with said optic body and defining an integral portion of said intraocular lens; characterised in that
said integral haptic portion is formed of a single piece of multi-axially stretched material selected from the group consisting of polymethylmethacrylate and copolymers thereof;
said multi-axially stretched material has an elongation in the direction of stretching in the range of from twenty percent to sixty-five percent along at least two mutually angulated and generally coplanar axes of elongation as determined by deformation of said material from its un-stretched condition, and said material is oriented relative to said plane of said optic body with said axes of elongation of said material being generally parallel with said plane of said optic body to provide oriented polymer chains in said haptic portion of said intraocular lens which has increased tensile strength and flexibility in comparison with un-stretched material.

2. The intraocular lens of claim 1 wherein said material is stretched to provide an elongation of forty percent.

3. The intraocular lens of claim 1 wherein said lens includes a pair of integral haptic portions both of which are formed of a single piece of said material.

4. A method of making an intraocular lens comprising the steps of:
stretching a sheet of material selected from the class of materials including polymethylmethacrylate and co-polymers thereof along at least two different and mutually angulated axes disposed in the plane of the sheet to provide highly oriented polymer chains within said material and increased strength and flexibility for said material;
selecting a blank from a section of said stretched sheet material which is spaced from edges thereof; and
forming from said blank of material an intraocular lens including a transparent optic body defining a plane which is disposed generally in the plane defined by said mutually angulated axes of stretching and which includes at least one integral haptic.

5. The method of claim 4 including the step of polishing said sheet prior to said stretching to minimize irregularities of the surfaces thereof.

6. The method of claim 4 wherein said step of stretching said sheet comprises stretching said sheet along at least four different and mutually angulated axes.

7. The method of claim 4 wherein said step of stretching said sheet includes the steps of:
wrapping said sheet in a protective cover;
heating said wrapped sheet;
stretching said heated and wrapped sheet; and uniformly cooling said stretched sheet.

8. The method of claim 4 further including the steps of:
selecting a thickness for said sheet before stretching thereof of from about 4 mm (0.125 inch) to about 25 mm (1 inch); and
stretching said sheet by an amount in the range of from about twenty percent to about sixty-five percent.

9. The method of claim 8 including the steps of:
selecting a thickness for said sheet before stretching of about 10 mm (3/8 inch); and
stretching said sheet by an amount providing an elongation along each stretching axis of about forty percent.

10. The method of claim 8 further including the step of exerting at least 1110 N (250 pounds) of tensile force upon said sheet to effect said stretching thereof.

11. The method of claim 4 further including the step of:
selecting a thickness for said sheet of at least 25 mm (1 inch);
cutting a plug from said sheet after stretching thereof, which plug has a length equal to the thickness of said stretched sheet; and
slicing said plug transversely into sections each one of which provides a
blank for a respective intraocular lens.

## Patentansprüche

1. Intraokulare Linse, umfassend einen transparenten optischen Körper (10), der eine Ebene definiert, und wenigstens ein haptisches Teil (12, 14), das an dem optischen Körper befestigt ist und sich auswärts von dem optischen Körper erstreckt,
wobei das wenigstens eine haptische Teil integral mit dem optischen Körper ist und einen integralen Teil der intraokularen Linse definiert;
dadurch **gekennzeichnet,** daß
der integrale haptische Teil von einem einzigen Stück von multiaxial gestrecktem Material gebildet ist, das aus der aus Polymethylmethacrylat und Copolymeren hiervon bestehenden Gruppe ausgewählt ist;
das multiaxial gestreckte Material eine Dehnung in der Richtung des Streckens in dem Bereich von zwanzig Prozent bis fünfundsechzig Prozent längs wenigstens zweier gegeneinander gewinkelter und generell koplanarer Dehnungsachsen hat, wie sie durch Deformation des Materials aus seinem ungestreckten Zustand bestimmt sind, und das Material relativ zu der Ebene des optischen Körpers ausgerichtet ist, wobei die Dehnungsachsen des Materials generell parallel zu der Ebene des optischen Körpers sind, um ausgerichtete Polymerketten in dem haptischen Teil der intraokularen Linse vorzusehen, welches im Vergleich zu ungestrecktem Material erhöhte Zugfestigkeit und Flexibilität hat.

2. Intraokulare Linse nach Anspruch 1, worin das Material so gestreckt ist, daß eine Dehnung von vierzig Prozent vorgesehen ist.

3. Intraokulare Linse nach Anspruch 1, worin die Linse ein Paar von integralen haptischen Teilen aufweist, welche beide aus einem einzigen Stück des Materials ausgebildet sind.

4. Verfahren zum Herstellen einer intraokularen Linse, umfassend die Schritte des:
Streckens einer Platte bzw. Dünnplatte von Material, das aus der Klasse von Materialien ausgewählt ist, die Polymethylmethacrylat und Copolymere hiervon umfaßt, längs wenigstens zweier unterschiedlicher und gegenseitig gewinkelter Achsen, die in der Ebene der Platte bzw. Dünnplatte angeordnet sind, um in hohem Maße ausgerichtete Polymerketten innerhalb des Materials und erhöhte Festigkeit und Flexibilität für das Material vorzusehen;
Auswählens eines Rohteils von einem Abschnitt des gestreckten Platten- bzw. Dünnplattenmaterials, welcher von den Rändern desselben beabstandet ist; und
Ausbildens einer intraokularen Linse aus dem Materialrohteil, die einen transparenten optischen Körper aufweist, der eine Ebene definiert, welche generell in der Ebene angeordnet ist, die durch die gegeneinander gewinkelten Achsen des Streckens definiert ist und welche wenigstens ein integrales haptisches Teil umfaßt.

5. Verfahren nach Anspruch 4, umfassend den Schritt des Polierens der Platte bzw. Dünnplatte vor dem Strecken, um Unregelmäßigkeiten der Oberflächen derselben zu minimieren.

6. Verfahren nach Anspruch 4, worin der Schritt des Streckens der Platte bzw. Dünnplatte das Strecken der Platte bzw. Dünnplatte längs wenigstens vier unterschiedlicher und gegeneinander gewinkelter Achsen umfaßt.

7. Verfahren nach Anspruch 4, worin der Schritt des Streckens der Platte bzw. Dünnplatte die Schritte des:
Umwickelns bzw. Einwickelns der Platte bzw. Dünnplatte in einer schützenden Abdeckung;
Erhitzens der um- bzw. eingewickelten Platte bzw. Dünnplatte;
Streckens der erhitzten und um- bzw. eingewickelten Platte bzw. Dünnplatte; und
gleichförmigen Kühlens der gestreckten Platte bzw. Dünnplatte;
umfaßt.

8. Verfahren nach Anspruch 4, weiter umfassend die Schritte des:
Auswählens einer Dicke für die Platte bzw. Dünnplatte vor dem Strecken derselben von etwa 4 mm (0.125 Zoll) bis etwa 25 mm (1 Zoll); und
Streckens der Platte bzw. Dünnplatte um einen Betrag in dem Bereich von etwa zwanzig Prozent bis etwa fünfundsechzig Prozent.

9. Verfahren nach Anspruch 8, umfassend die Schritte des:
Auswählens einer Dicke für die Platte bzw. Dünnplatte vor dem Strecken von etwa 10 mm (3/8 Zoll); und
Streckens der Platte bzw. Dünnplatte um einen Betrag, der eine Dehnung längs jeder Streckachse von etwa vierzig Prozent liefert.

10. Verfahren nach Anspruch 8, weiter umfassend den Schritt des Ausübens von wenigstens 1110 N (250 Pound) Zugkraft auf die Platte bzw. Dünnplatte zum Bewirken des Streckens derselben.

11. Verfahren nach Anspruch 4, weiter umfassend den Schritt des:
Auswählens einer Dicke für die Platte bzw. Dünnplatte von wenigstens 25 mm (1 Zoll);
Schneidens eines Klotzes aus der Platte bzw. Dünnplatte nach dem Strecken derselben, welcher Klotz eine Länge hat, die gleich der Dicke der gestreckten Platte bzw. Dünnplatte ist; und
Querdurchschneidens des Klotzes in Abschnitte, von denen jeder einen Rohling für eine jeweilige intraokulare Linse liefert.

## Revendications

1. Lentille intra-oculaire comprenant un corps optique transparent (10) définissant un plan et au moins un élément haptique (12, 14) fixé audit corps optique, et s'étendant vers l'extérieur à partir de ce corps optique,
ledit élément haptique présent en au moins un exemplaire faisant partie intégrante dudit corps optique et définissant une portion intégrale de ladite lentille intraoculaire ; caractérisée en ce que
ladite portion haptique intégrale est formée d'une seule pièce d'une matière à étirage multiaxial choisie dans le groupe consistant en un polymère de méthacrylate de méthyle et ses copolymères ;
ladite matière à étirage multiaxial ayant un allongement dans la direction d'étirage compris dans l'intervalle de 20 % à 65 % le long d'au moins deux axes d'allongement faisant mutuellement un angle et généralement coplanaires, déterminés par la déformation de ladite matière à partir de son état non étiré, et ladite matière étant orientée par rapport audit plan dudit corps optique avec lesdits axes d'allongement de ladite matière généralement parallèles audit plan dudit corps optique pour engendrer des chaînes polymériques orientées dans ladite portion haptique de ladite lentille intra-oculaire, présentant une résistance à la traction et une flexibilité accrues comparativement à la matière non étirée.

2. Lentille intra-oculaire suivant la revendication 1, dans laquelle ladite matière est étirée pour parvenir à un allongement de 40 %.

3. Lentille intra-oculaire suivant la revendication 1, qui comprend une paire de portions haptiques intégrales qui sont toutes deux formées d'une seule pièce de ladite matière.

4. Procédé de production d'une lentille intraoculaire, comprenant les étapes consistant :
à étirer une feuille d'une matière choisie dans la catégorie de matières comprenant un polymère de méthacrylate de méthyle et ses copolymères le long d'au moins deux axes différents et faisant mutuellement un angle, disposés dans le plan de la feuille, pour engendrer des chaînes polymériques hautement orientées à l'intérieur de ladite matière et une résistance mécanique et une flexibilité accrues de ladite matière :
à choisir une pièce dans une section de ladite matière sous forme de feuille étirée qui est espacée de ses bords ; et
à former à partir de ladite pièce de matière une lentille intra-oculaire comprenant un corps optique transparent définissant un plan qui est disposé généralement dans le plan défini par lesdits axes d'étirement faisant mutuellement un angle et qui comprend au moins un élément haptique intégral.

5. Procédé suivant la revendication 4, comprenant l'étape de polissage de la feuille avant l'étirage pour réduire au minimum les irrégularités des surfaces de cette feuille.

6. Procédé suivant la revendication 4, dans lequel l'étape d'étirage de la feuille comprend l'étirage de ladite feuille le long d'au moins quatre axes différents et faisant mutuellement des angles.

7. Procédé suivant la revendication 4, dans lequel l'étape d'étirage de la feuille comprend les étapes consistant :
à emballer ladite feuille dans une enveloppe protectrice ;
à chauffer ladite feuille emballée ;
à étirer ladite feuille chauffée et emballée ; et à refroidir uniformément ladite feuille étirée.

8. Procédé suivant la revendication 4, comprenant en outre les étapes consistant :
à choisir une épaisseur pour ladite feuille avant son étirage d'environ 4 mm (0,125 inch) à environ 25 mm (1 inch) ; et
à étirer ladite feuille à un degré d'étirage compris dans l'intervalle d'environ 20 % à environ 65 %.

9. Procédé suivant la revendication 8, comprenant les étapes consistant :
à choisir une épaisseur pour la feuille avant étirage d'environ 10 mm (3/8 inch) ; et
à étirer ladite feuille à un degré d'étirage provoquant un allongement le long de chaque axe d'étirage d'environ 40 %.

10. Procédé suivant la revendication 8, comprenant en outre l'étape consistant à exercer une force de traction d'au moins 1110 N (250 lb) sur la feuille pour effectuer l'étirage de cette feuille.

11. Procédé suivant la revendication 4, comprenant en outre les étapes consistant :
à choisir une épaisseur pour ladite feuille d'au moins 25 mm (1 inch) ;
à découper une pastille dans ladite feuille après son étirage, pastille qui a une longueur égale à l'épaisseur de ladite feuille étirée ; et
à couper en tranches ladite pastille transversalement en sections dont chacune fournit une pièce pour une lentille intra-oculaire respective.
